# EUROPEAN PATENT APPLICATION

(11) **EP 4 140 453 A1**
(43) Date of publication of application: **01.03.2023**
(21) Application number: 22191695.0
(22) Date of filing: 23.08.2022
(51) Int. Cl.: A61F 2/64, A61F 2/74

(54) **ARTIFICIAL KNEE JOINT AND PROSTHETIC LIMB**

(30) Priority: 25.08.2021 JP 2021137159
(71) Applicant: Nabtesco Corporation, Tokyo 102-0093 (JP)
(72) Inventor: Okuda, Masahiko, Chiyoda-ku, Tokyo (JP)
(74) Representative: Grünecker Patent- und Rechtsanwälte PartG mbB

(57) **Abstract**

A multi-articulated link knee joint includes a thigh joint part (32) provided on a thigh part side, a lower leg part (12) rotatably connected to the thigh joint part (32) via at least one rotation shaft, a cylinder (62) fixed to the lower leg part (12) and filled with a working fluid, a piston (66) provided movably between a first cylinder wall (61) of the lower leg part (12) side and a second cylinder wall (63) of the thigh joint part (32) side in the cylinder (62), a piston rod (64) extending from the piston (66) toward the thigh joint part (32) and penetrating the second cylinder wall (69), and a connecting rod (65) that connects the piston rod (64) to the thigh joint part (32).

## Description

The present invention relates to an artificial knee joint and a prosthetic limb.

### 2. Description of the Related Art

JP H7-308332 A discloses an artificial knee joint or a prosthetic limb worn by a person who has lost a leg due to injury or illness, which includes an air cylinder that swings back and forth. In the air cylinder pivotally supported by a lower leg part, air as a working fluid is sealed therein and a piston is movably provided. Also, a piston rod extending from the piston is coupled to a thigh joint part. When the thigh joint part and the lower leg part relatively rotate according to bending and stretching of the artificial knee joint, the piston moves in the air cylinder and the entire air cylinder rotates around the axis of the lower leg part and swings back and forth.

In the structure of JP H7-308332 A, since the air cylinder swings back and forth, the substantial dimension of the artificial knee joint in the front-rear direction becomes long. In addition, since the space on the swing path of the air cylinder becomes a dead space, in particular, in the electronically controlled artificial knee joint, it is necessary to mount many functional components such as a sensor, a motor, a control board, and a battery in other locations, which causes an increase in size of the artificial knee joint.

The present invention addresses the above-described issue and a purpose thereof is to provide an artificial knee joint and a prosthetic limb that can be configured compactly.

In order to solve the above problems, an artificial knee joint according to an aspect of the present invention includes: a thigh joint part provided on a thigh part side; a lower leg part rotatably connected to the thigh joint part via at least one rotation shaft; a cylinder fixed to one of the thigh joint part and the lower leg part and filled with a working fluid; a piston provided movably between the thigh joint part side and the lower leg part side in the cylinder; a cylinder wall provided in the cylinder and defining a motion limit of the other side of the thigh joint part and the lower leg part of the piston; a piston rod extending from the piston toward the other of the thigh joint part and the lower leg part and penetrating the cylinder wall; and a connecting rod that connects the piston rod to the other of the thigh joint part and the lower leg part and is connected to the piston rod at a connecting part that is separated by a predetermined distance or more from the other end of the other of the thigh joint part and the lower leg part on the piston rod toward one end on the piston side. When the piston is at the limit position on one side of the thigh joint part and lower leg part, the connecting part of the connecting rod and the piston rod is located between the cylinder wall and the piston.

In this aspect, since the piston rod is connected to the thigh joint part or lower leg part via the connecting rod, it is possible to suppress the swinging of the piston rod and the cylinder and to reduce the dimension of the artificial knee joint in the front-rear direction. In addition, since the connecting rod is connected to the connecting part separated from the other end of the piston rod, the dimension of the artificial knee joint in the vertical direction can also be reduced. Therefore, the artificial knee joint can be configured compactly.

Another aspect of the present invention also relates to an artificial knee joint. This artificial knee joint includes: a thigh joint part provided on a thigh part side; a lower leg part rotatably connected to the thigh joint part via at least one rotation shaft; a rotational resistance applicator including: a cylinder fixed to one of the thigh joint part and the lower leg part and filled with a working fluid; a piston provided movably between the thigh joint part side and the lower leg part side in the cylinder; a cylinder wall provided in the cylinder and defining a motion limit of the piston; and a rod part connecting the piston and the other of the thigh joint part and the lower leg part, the rotational resistance applicator being structured to apply a resistance by the working fluid according to a position of the piston in the cylinder to relative rotation of the thigh joint part and the lower leg part; and functional components that are stored inside the cylinder wall and control the resistance applied by the rotational resistance applicator according to a phase of walking of a user.

In this aspect, since the functional components that control the resistance applied by the rotational resistance applicator according to the phase of walking of the user of the artificial knee joint are stored inside the cylinder wall, the artificial knee joint can be made compact.

Still another aspect of the present invention is a prosthetic limb. This prosthetic limb includes: a thigh part; a thigh joint part provided on the thigh part side; a lower leg part rotatably connected to the thigh joint part via at least one rotation shaft; a cylinder fixed to one of the thigh joint part and the lower leg part and filled with a working fluid; a piston provided movably between the thigh joint part side and the lower leg part side in the cylinder; a cylinder wall provided in the cylinder and defining a motion limit of the other side of the thigh joint part and the lower leg part of the piston; a piston rod extending from the piston toward the other of the thigh joint part and the lower leg part and penetrating the cylinder wall; and a connecting rod that connects the piston rod to the other of the thigh joint part and the lower leg part and is connected to the piston rod at a connecting part that is separated by a predetermined distance or more from the other end of the other side of the thigh joint part and the lower leg part on the piston rod toward one end on the piston side. When the piston is at the limit position on one side of the thigh joint part and lower leg part, the connecting part of the connecting rod and the piston rod is located between the cylinder wall and the piston.

Another aspect of the present invention also relates to a prosthetic limb. This prosthetic limb includes: a thigh part, a thigh joint part provided on the thigh part side; a lower leg part rotatably connected to the thigh joint part via at least one rotation shaft; a rotational resistance applicator including: a cylinder fixed to one of the thigh joint part and the lower leg part and filled with a working fluid; a piston provided movably between the thigh joint part side and the lower leg part side in the cylinder; a cylinder wall provided in the cylinder and defining a motion limit of the piston; and a rod part connecting the piston and the other of the thigh joint part and the lower leg part, the rotational resistance applicator being structured to apply a resistance by the working fluid according to a position of the piston in the cylinder to relative rotation of the thigh joint part and the lower leg part; and functional components that are stored inside the cylinder wall and control the resistance applied by the rotational resistance applicator according to a phase of walking of a user.

Optional combinations of the aforementioned constituting elements, and implementations of the disclosure in the form of methods, apparatuses, systems, recording mediums, and computer programs may also be practiced as additional modes of the present invention.
Fig. 1 is a side view of a multi-articulated link knee joint in a swing cylinder type;
Fig. 2 is a schematic cross-sectional view of the multi-articulated link knee joint in the swing cylinder type;
Figs. 3A to 3D illustrate a manner in which a knee part is bent in the multi-articulated link knee joint;
Fig. 4 is a functional block diagram of a control device;
Fig. 5 is a side view of the multi-articulated link knee joint in a fixed cylinder type;
Figs. 6A and 6B are side views of the multi-articulated link knee joint in the fixed cylinder type each having a configuration for solving the problem in Fig. 5;
Figs. 7A and 7B are schematic diagrams of the multi-articulated link knee joint in the fixed cylinder type;
Figs. 8A and 8B illustrate an arrangement example of other functional components;
Fig. 9 schematically illustrates a cylinder device;
Figs. 10A1, 10A2, 10B1, and 10B2 schematically illustrate a manner in which the cylinder device applies rotational resistance to bending and stretching motions of the multi-articulated link knee joint;
Figs. 11A and 11B illustrate examples in which the functional components of the multi-articulated link knee joint are stored inside a cylinder wall; and
Figs. 12A and 12B illustrate examples in which a stepwise control mechanism of bend resistance is stored inside the cylinder wall.

The invention will now be described by reference to the preferred embodiments. This does not intend to limit the scope of the present invention, but to exemplify the invention.

There are various types of artificial knee joints, and they are roughly classified into an active type and a passive type. The passive type is further classified into an electronically controlled type and a mechanical type. In the active type, the knee is actively driven to be bent and stretched by an actuator such as a motor. In the electronically controlled passive type, the rotational resistance of the knee is controlled by an electronically controlled air cylinder, hydraulic cylinder, or the like according to the bending and stretching motion of the user such as walking. In the mechanical passive type, the bending and stretching motion of the user such as walking is assisted by an air cylinder, a hydraulic cylinder, or the like in which the amount and pressure of the working fluid are adjusted in advance. The present invention is applicable to any type of artificial knee joint as long as it is consistent with the description of the present embodiment described below. In particular, among the configurations described regarding the electronically controlled artificial knee joint in the present embodiment, a mechanical configuration not premised on the rotational resistance control according to the phase of walking of the user is also applicable to a mechanical knee joint.

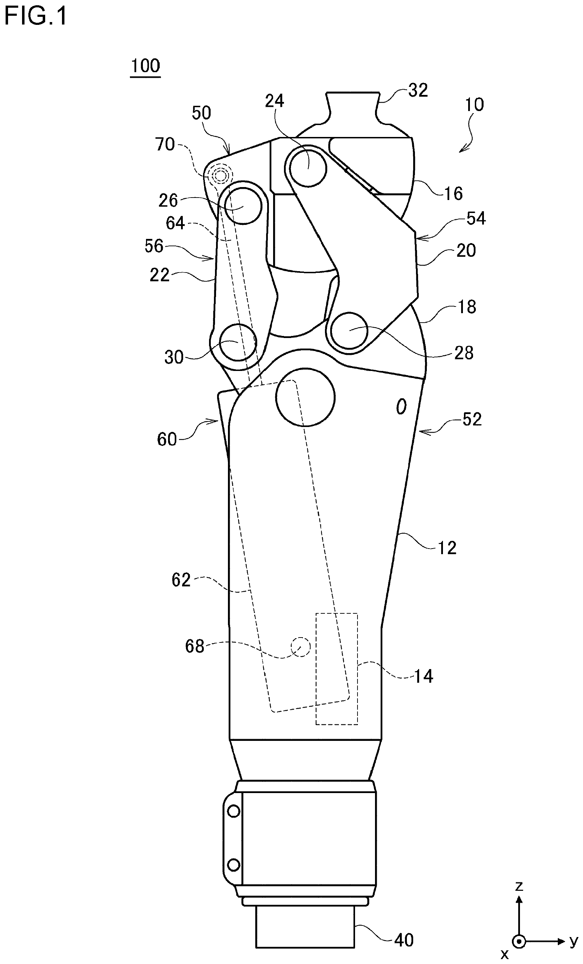

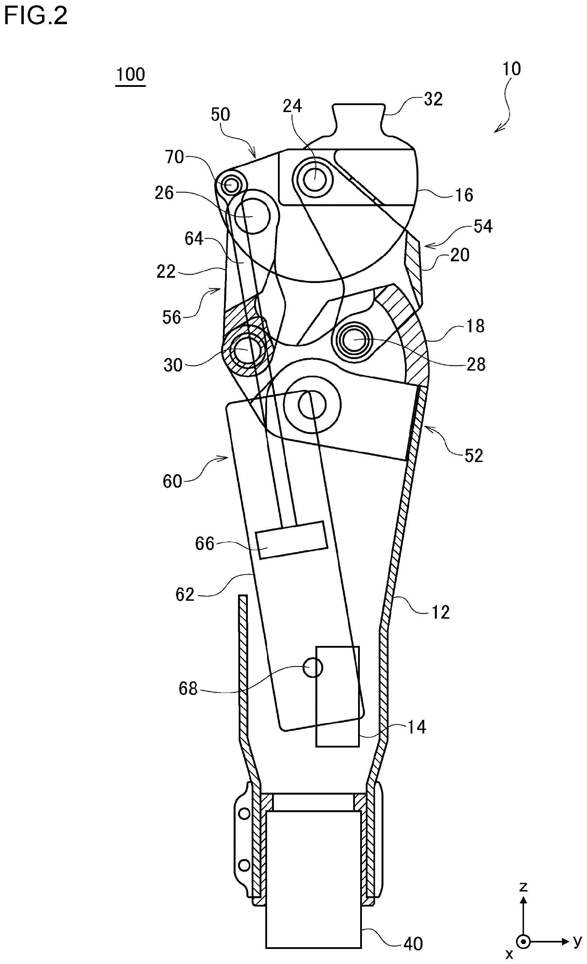

First, an outline of the artificial knee joint will be described. Fig. 1 is a side view of a multi-articulated link knee joint 100 in a swing cylinder type. Fig. 2 is a schematic cross-sectional view of the multi-articulated link knee joint 100 in the swing cylinder type. In the following description, in the xyz orthogonal coordinate system illustrated in each drawing, when provided that the direction parallel to the x-axis is the left-right direction, a positive direction of the x axis is referred to as "left" and a negative direction of the x axis is referred to as "right", when provided that the direction parallel to the y-axis is the front-rear direction, a positive direction of the y axis is referred to as "front" and a negative direction of the y axis is referred to as "rear", and when provided that the direction parallel to the z-axis is the vertical direction, a positive direction of the z axis is referred to as "upper" and a negative direction of the z axis is referred to as "lower".

The multi-articulated link knee joint 100 includes a knee part 10. The knee part 10 is bent by a multi-articulated link mechanism having a plurality of link parts. The multi-articulated link mechanism includes four link parts of an upper link part 50, a lower link part 52, a front link part 54, and a rear link part 56. In the present description, a link and a part that is fixed to the link so as to move together with the link are collectively referred to as a "link part". The upper link part 50 includes an upper link 16 and a thigh joint part 32. The lower link part 52 includes a lower link 18 and a lower leg part 12. The front link part 54 includes a front link 20, and the rear link part 56 includes a rear link 22.

The upper link 16 is provided with a first shaft 24 and a second shaft 26 as rotation shafts, and the lower link 18 is provided with a third shaft 28 and a fourth shaft 30 as rotation shafts. Each shaft is rotatably provided with the axial direction being parallel to the x axis. The front link 20 is attached to the ends of the first shaft 24 and the third shaft 28. The rear link 22 is attached to the ends of the second shaft 26 and the fourth shaft 30. The upper link 16 is supported by the front link 20 and the rear link 22 and rotates with respect to the lower link 18. In this manner, the upper link 16 to which the thigh joint part 32 is fixed and the lower link 18 to which the lower leg part 12 is fixed are rotatably connected via a plurality of rotation shafts (that is, a pair of the first shaft 24 and the third shaft 28, or a pair of the second shaft 26 and the fourth shaft 30) and a pair of left and right links (that is, two front links 20 or two rear links 22) connecting both ends of the plurality of rotation shafts to each other.

The thigh joint part 32 protruding from the upper link 16 is connected to a socket attached to the thigh part of the user. An angle formed by the direction in which thigh joint part 32 protrudes and the z axis is defined as a bending angle or a knee angle of knee part 10. The bending angle of the knee part 10 can be measured by a knee angle sensor, an inertial sensor, or the like (not illustrated). In Figs. 1 and 2, the bending angle is 0 degrees, and the knee part 10 is fully stretched. The lower leg part 12 is formed in a tubular shape and is fixed below the lower link 18. Furthermore, a foot joint part 40 connected to a foot part constituting the prosthetic limb is provided below the lower leg part 12.

The multi-articulated link knee joint 100 includes a cylinder device 60 as an assisting driver that assists the motion of the knee part 10. The cylinder device 60 includes, for example, an air cylinder or a hydraulic cylinder. The cylinder device 60 includes a cylinder 62, a piston rod 64 movable with respect to the cylinder 62, and a piston 66 to which the piston rod 64 is fixed and that is movably housed inside the cylinder 62. The cylinder device 60 is provided so as to connect the upper link part 50 and the lower link part 52. Specifically, the cylinder 62 is rotatably supported by a lower shaft 68 provided in the lower leg part 12 of the lower link part 52, and the piston rod 64 is rotatably supported by an upper shaft 70 provided in the upper link 16 of the upper link part 50. With this configuration, the cylinder device 60 moves or swings in the front-rear direction about the lower shaft 68 according to the rotation of the upper link part 50.

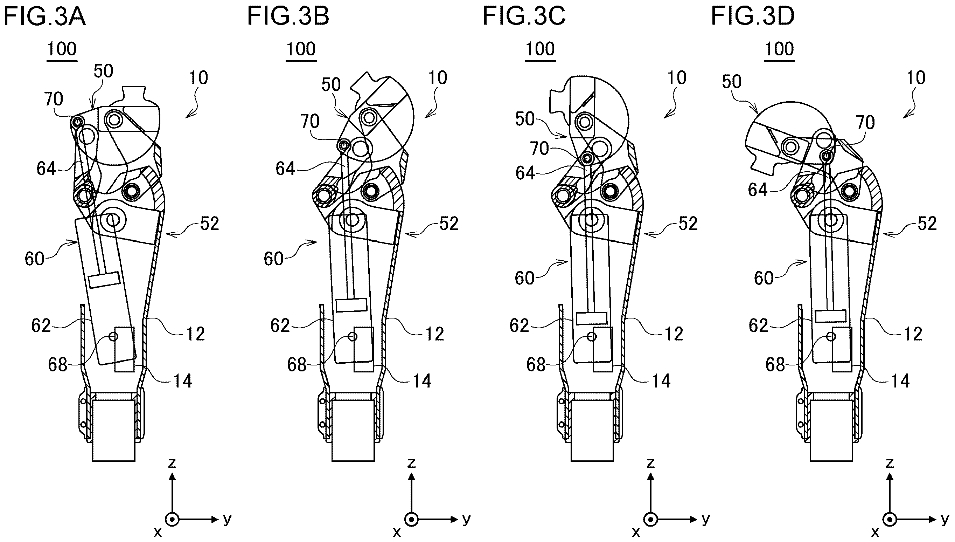

Figs. 3A to 3D illustrate a manner in which a knee part 10 is bent in the multi-articulated link knee joint 100. The bending angles of the knee part 10 illustrated in Figs. 3A to 3D are 0 degrees, 45 degrees, 90 degrees, and 160 degrees, respectively. When the bending angle increases, the front link 20 and the rear link 22 intersect. The upper link 16 rotates while moving backward with respect to the lower link 18. The knee part 10 is bent similarly to the knee joint of the living body by the rotation of the upper link 16.

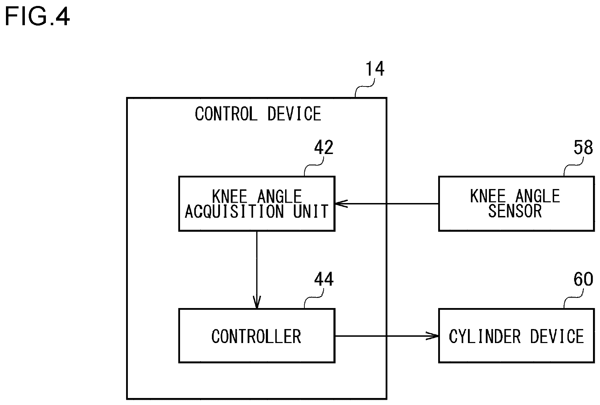

The multi-articulated link knee joint 100 further includes a control device 14. The control device 14 is housed inside the lower leg part 12, and controls the cylinder device 60 according to the bending angle of the knee part 10 measured by a knee angle sensor, an inertial sensor, or the like. Fig. 4 is a functional block diagram of the control device 14. Each functional block illustrated here can be realized by an arithmetic element such as a CPU of a computer in terms of hardware, and is realized by a computer program or the like in terms of software.

The control device 14 includes a knee angle acquisition unit 42 and a controller 44. The knee angle acquisition unit 42 acquires the bending angle of the knee part 10 from a knee angle sensor 58 such as a knee angle sensor or an inertial sensor. The controller 44 controls the cylinder device 60 according to the knee angle acquired by the knee angle acquisition unit 42 to assist the motion of the knee part 10. For example, when the bending angle is close to 0 degrees, the controller 44 controls the cylinder device 60 so as to limit the rotation of the third shaft 28, thereby preventing the knee instability in which the knee part 10 is bent against the intention of the user. In addition, the controller 44 controls the cylinder device 60 to allow the rotation of the third shaft 28 according to the change to be taken in the knee angle during the swing period when the bending angle changes, such as when walking. With this configuration, the lower leg part 12 can swing according to the swinging of each foot at the time of walking, so that the user can walk comfortably.

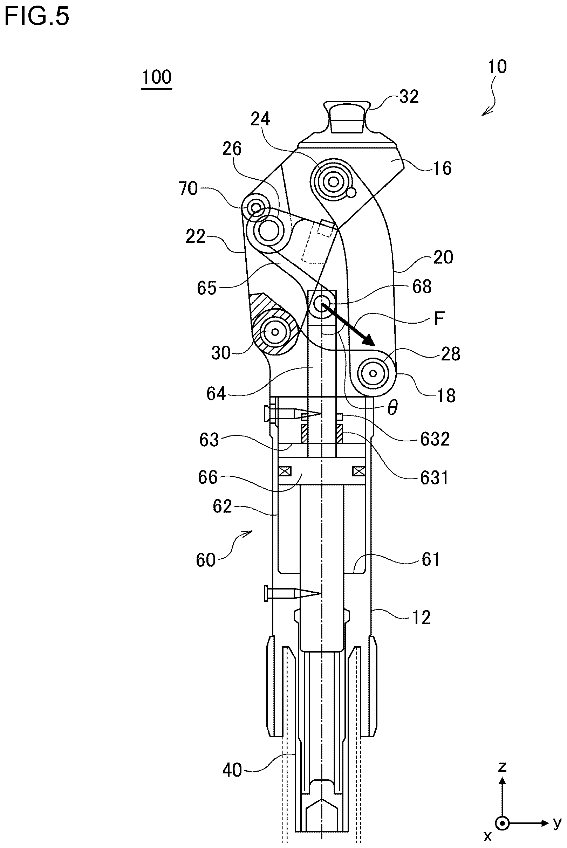

Fig. 5 is a side view of a multi-articulated link knee joint 100 in a fixed cylinder type. Constituent elements similar to those of the multi-articulated link knee joint 100 in the swing cylinder type in Figs. 1 and 2 are denoted by the same reference numerals, and description thereof is omitted.

A cylinder device 60 such as an air cylinder or a hydraulic cylinder that assists the motion of the knee part 10 includes a cylinder 62 that is fixed to the lower leg part 12 and in which air or oil as a working fluid is sealed therein, a piston 66 that is provided in the cylinder 62 so as to be movable in the vertical direction or the z-axis direction between a first cylinder wall 61 on the lower leg part 12 side and a second cylinder wall 63 on the thigh joint part 32 side, and a piston rod 64 that extends upward from the piston 66 toward the thigh joint part 32 and penetrates the second cylinder wall 63. On the inner peripheral surface of the second cylinder wall 63 on which the outer peripheral surface of the piston rod 64 slides with the motion of the piston 66 in the vertical direction, a bearing 631 that pivotally supports the piston rod 64 and a seal member 632 such as an O-ring that seals the cylinder 62 so as not to leak the working fluid are provided. Here, the first cylinder wall 61 and the second cylinder wall 63 define each of the upward and downward motion limits of the piston 66.

The piston rod 64 constitutes a rod part connecting the piston 66 and the thigh joint part 32 together with the connecting rod 65. Specifically, the upper end of the connecting rod 65 is rotatably supported by the upper shaft 70 provided on the upper link 16, and the lower end of the connecting rod 65 is rotatably supported by the lower shaft 68 as a connecting part provided at the upper end of the piston rod 64 on the thigh joint part 32 side. With this configuration, the connecting rod 65 moves or swings in the front-rear direction about the lower shaft 68 according to the rotation of the upper link 16 and thigh joint part 32. On the other hand, unlike the multi-articulated link knee joint 100 in the swing cylinder-type in Figs. 1 and 2, since the cylinder 62, which is the main body of the cylinder device 60, is integrally formed with the lower leg part 12, the cylinder device 60 does not swing in the front-rear direction even when the thigh joint part 32 rotates. Therefore, the dimension in the front-rear direction of the multi-articulated link knee joint 100 can be reduced as compared with that in Figs. 1 and 2. However, providing the connecting rod 65 further above the piston rod 64 may increase the vertical dimension of the multi-articulated link knee joint 100.

In addition, as indicated by an arrow from the lower shaft 68, as the connecting part of the piston rod 64 and the connecting rod 65 positioned upward, the angle θ formed by the force F exerted by the connecting rod 65 on the distal end of the piston rod 64 and the vertical direction becomes larger, so that a large force Fsinθ in the front-rear direction or the lateral direction is applied to the piston rod 64. As a result, when an excessive load is applied to the bearing member such as the second cylinder wall 63 that axially supports the piston rod 64 from the side, wear of the bearing 631 and the piston rod 64 is promoted, leading to deterioration of durability of the multi-articulated link knee joint 100.

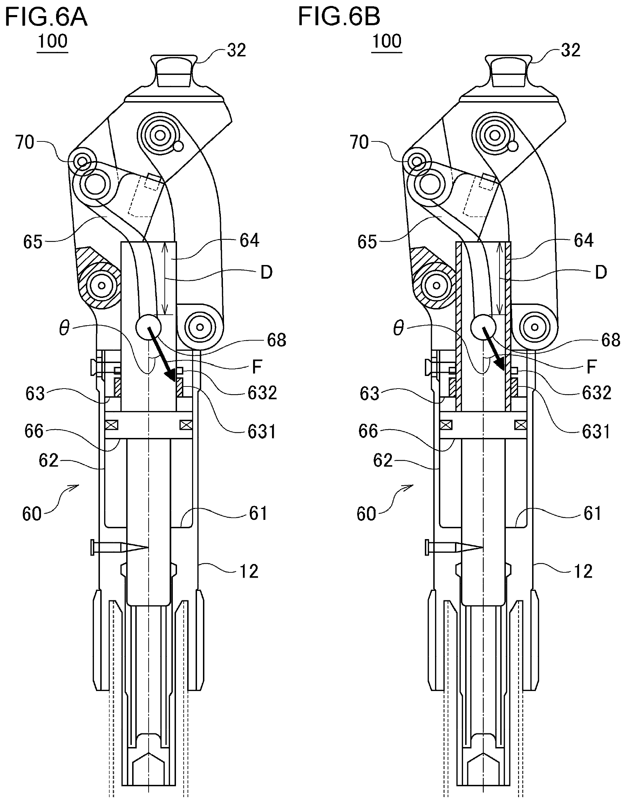

Figs. 6A and 6B are side views of the multi-articulated link knee joint 100 in the fixed cylinder type each having a configuration for solving each problem described above. In each of Figs. 6A and 6B, the connecting part 68 of the piston rod 64 and the connecting rod 65 is provided at a position separated by a predetermined distance D from the upper end of the piston rod 64 on the thigh joint part 32 side toward the lower end on the piston 66 side. In Fig. 6A, the connecting part 68 is integrally formed inside the piston rod 64 formed in a columnar shape, and in Fig. 6B, the connecting part 68 is provided inside the tubular part of the piston rod 64 formed in a tubular shape. The distance D of the connecting part 68 from the upper end of the piston rod 64 can be arbitrarily set as long as it is significant with respect to the overall length of the piston rod 64. For example, provided that the distance from the upper end of the piston rod 64 to the upper surface of the piston 66 is "100", the distance D from the upper end of the piston rod 64 to the connecting part 68 can be set to, for example, "5 or more", "10 or more", "25 or more", "50 or more", "75 or more", "100", or the like.

With such a configuration, since the cylinder device 60 does not swing in the front-rear direction as in Fig. 5, not only the dimension in the front-rear direction of the multi-articulated link knee joint 100 can be reduced, but also the dimension in the vertical direction of the multi-articulated link knee joint 100 can be reduced since the connecting rod 65 is connected to the connecting part 68 that is separated from the upper end of the piston rod 64. Therefore, the multi-articulated link knee joint 100 can be configured compactly. As the distance D is larger, the dimension in the vertical direction of the multi-articulated link knee joint 100 can be reduced, but it is necessary to increase the dimension in the front-rear direction of the piston rod 64 in order to allow the connecting rod 65 to swing in the front-rear direction in the piston rod 64. Therefore, the distance D is preferably set as large as possible within a range in which the dimension of the piston rod 64 in the front-rear direction is allowable.

In addition, since the connecting part 68 of the piston rod 64 and the connecting rod 65 is located below as compared with Fig. 5, the angle θ formed by the force F exerted by the connecting rod 65 on the piston rod 64 (acting on the straight line connecting the upper shaft 70 and the connecting part 68) and the vertical direction becomes smaller, and the force Fsinθ in the front-rear direction or the lateral direction applied to the piston rod 64 also becomes smaller. As a result, the load on the bearing 631 pivotally supporting the piston rod 64 from the side is reduced, and wear is suppressed, so that deterioration of durability of the multi-articulated link knee joint 100 can be prevented.

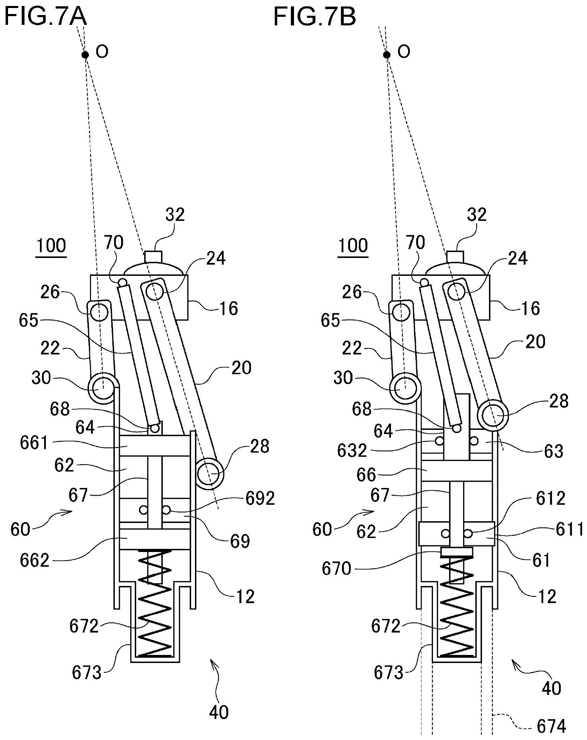

Figs. 7A and 7B are schematic diagrams of the multi-articulated link knee joint 100 in the fixed cylinder type. Fig. 7B illustrates a multi-articulated link knee joint 100 having a configuration similar to that of Figs. 6A and 6B, and Fig. 7A illustrates a comparative example. In Fig. 7B, the second cylinder wall 63 (bearing 631 is not illustrated) on the thigh joint part 32 side or the upper side in the cylinder device 60 is integrally formed with the cylinder 62, and the first cylinder wall 61 on the lower leg part 12 side or the lower side in the cylinder device 60 can be attached to and detached from the cylinder 62 from the lower side. Similarly to the piston rod 64 extending upward from the piston 66 penetrating the second cylinder wall 63, the lower piston rod 67 extending downward from the piston 66 penetrates the first cylinder wall 61. On the inner peripheral surface of the first cylinder wall 61 on which the outer peripheral surface of the lower piston rod 67 slides with the motion of the piston 66 in the vertical direction, a bearing (not illustrated) that pivotally supports the lower piston rod 67 and a seal member 612 such as an O-ring that seals the cylinder 62 so as not to leak the working fluid are provided.

The lower piston rod 67 constituting the rod part together with the piston rod 64 and the connecting rod 65 includes a flange part 670 protruding in the radial direction (the x-axis direction and the y-axis direction) at the tip penetrating the first cylinder wall 61 from the upper side to the lower side. The diameter of the flange part 670 is larger than the diameter of the cross section surrounded by the inner peripheral surface of the first cylinder wall 61, and when the piston 66 moves upward, the upper surface of the flange part 670 abuts against the lower surface of the first cylinder wall 61, so that the piston 66 is stopped. In other words, the flange part 670 defines an upper stroke length, which is a maximum upward motion amount of the piston 66.

An extension assist spring 672 that biases the lower piston rod 67 upward is attached to the lower surface of the flange part 670. The extension assist spring 672 urges the thigh joint part 32 via the lower piston rod 67, the piston 66, the upper piston rod 64, the connecting rod 65, and the upper link 16 in the clockwise direction, that is, the stretching direction of the multi-articulated link knee joint 100. Accordingly, when the multi-articulated link knee joint 100 is slightly bent, it is possible to naturally return to the stretched state by the biasing force of the extension assist spring 672. The lower end of the extension assist spring 672 whose upper end biases the lower surface of the flange part 670 upward biases the bottom surface of a tubular holder 673 that houses the extension assist spring 672 downward. An outer peripheral surface of the holder 673 constitutes the foot joint part 40, and an attachment pipe 674 (shown in broken line) having a foot part (not illustrated) at a lower end is inserted from below, whereby the foot part is attached to lower leg part 12.

The comparative example of Fig. 7A is different from Fig. 7B mainly in the configuration of the cylinder device 60. The cylinder device 60 includes the cylinder 62, a central cylinder wall 69 integrally formed with the cylinder 62 and vertically defining the inside thereof, an upper piston 661 and a lower piston 662 that are connected and movably provided with the cylinder wall 69 interposed therebetween from above and below, the upper piston rod 64 fixed to an upper surface of the upper piston 661, and the lower piston rod 67 that penetrates the cylinder wall 69 and connects the upper piston 661 and the lower piston 662 to each other. On the inner peripheral surface of the cylinder wall 69 on which the outer peripheral surface of the lower piston rod 67 slides with the motion of the two pistons 661 and 662 in the vertical direction, a bearing (not illustrated) that pivotally supports the lower piston rod 67 and a seal member 692 such as an O-ring that seals the cylinder 62 so as not to leak the working fluid are provided. Here, the upper surface of the cylinder wall 69 defines a downward motion limit of the upper piston 661, and the lower surface of the cylinder wall 69 defines an upward motion limit of the lower piston 662.

The upper piston 661 forms an upper pressure chamber in which air or oil as a working fluid is sealed with the upper surface of the cylinder wall 69, and the lower piston 662 forms a lower pressure chamber in which air or oil as a working fluid is sealed with the lower surface of the cylinder wall 69. In addition, the lower piston 662 performs a function similar to that of the flange part 670 in Fig. 7B. Specifically, since the lower piston 662 is stopped by abutting the lower surface of the cylinder wall 69 when moving upward, the lower piston 662 defines an upper stroke length, which is a maximum upward motion amount (similarly, the upper piston 661 defines a downward stroke length, which is a maximum downward motion amount). The lower surface of the lower piston 662 is biased upward by the extension assist spring 672.

The upper piston rod 64 fixed to the upper surface of the upper piston 661 is a member that is short in the vertical direction, unlike the piston rod 64, which is long in the vertical direction in Figs. 5, 6A, 6B, and 7B. Therefore, the connecting part 68 of the piston rod 64 and the connecting rod 65 is provided close to the upper end of the piston rod 64. This is in contrast to Figs. 6A and 6B in which the connecting part 68 is provided at a position that is separated from the upper end of the piston rod 64 by the predetermined distance D.

In the configuration of Fig. 7A, the cylinder wall 69 is integrally formed with the cylinder 62, and it is necessary to insert the upper piston 661 and the lower piston 662 into the cylinder 62 respectively from above and below the cylinder wall. Therefore, functional components of the multi-articulated link knee joint 100 cannot be disposed in the space above the upper piston 661 and the space below the lower piston 662 except for simple mechanical components such as the extension assist spring 672. On the other hand, in the configuration of Fig. 7B, the upper second cylinder wall 63 is integrally formed with the cylinder 62, while the lower first cylinder wall 61 can be attached to and detached from the cylinder 62 from the lower side. When assembling such a cylinder device 60, a structured member in which the upper piston rod 64, the piston 66, the lower piston rod 67, and the first cylinder wall 61 are combined may be inserted from below the cylinder 62 (thereafter, attachment of the extension assist spring 672 and installation of the holder 673 are performed). Unlike the configuration of Fig. 7A, the space above the second cylinder wall 63 is not used at the time of assembling the cylinder device 60, so that the functional components of the multi-articulated link knee joint 100 can be disposed. A seal member 611 such as one or a plurality of O-rings is provided on the outer peripheral surface of the first cylinder wall 61 so that the working fluid in the cylinder 62 does not leak from between the outer peripheral surface of the assembled first cylinder wall 61 and the inner peripheral surface of the cylinder 62.

In Fig. 7B, as an example of a functional component, the third shaft 28 (rotation shaft) is provided above the second cylinder wall 63 on the thigh joint part 32 side. On the other hand, in Fig. 7A, the third shaft 28 cannot be provided above the upper piston 661. Regarding the arrangement of each rotation shaft of the multi-articulated link knee joint 100, since there is a required condition for ensuring stability that the instant center of rotation O, which is the intersection of the straight line connecting the first shaft 24 and the third shaft 28 at both ends of the front link 20 and the straight line connecting the second shaft 26 and the fourth shaft 30 at both ends of the rear link 22, must be above and behind the knee part 10, the third shaft 28 in Fig. 7A must be arranged in front of and below the upper piston 661. Therefore, the dimension in the front-rear direction of the multi-articulated link knee joint 100 in Fig. 7A increases. In the multi-articulated link knee joint 100 of Fig. 7B, since the lower first cylinder wall 61 is formed in an assembled manner, the third shaft 28 can be disposed by effectively utilizing the space above the upper second cylinder wall 63, so that the dimension in the front-rear direction can be reduced.

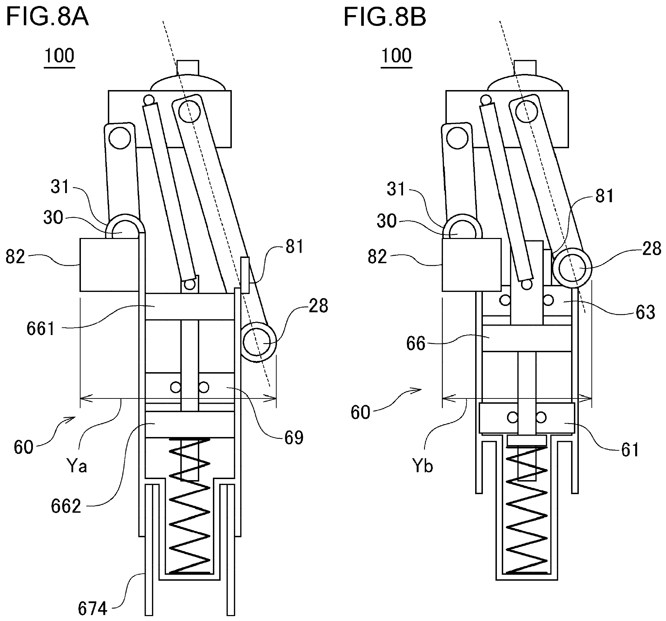

Figs. 8A and 8B illustrate arrangement examples of other functional components. The configurations of Figs. 8A and 8B are similar to the configurations of Figs. 7A and 7B, respectively. In Fig. 8A, similarly to Fig. 7A, since the functional components cannot be disposed in the space above the upper piston 661, the third shaft 28 and a sensor 81 are disposed in front of the cylinder device 60, and a bumper 82 is disposed behind the cylinder device 60. In contrast, in Fig. 8B, at least some of all the functional components can be provided above the second cylinder wall 63. Therefore, a dimension Yb in the front-rear direction of the multi-articulated link knee joint 100 in Fig. 8B can be made smaller than a dimension Ya in the front-rear direction of the multi-articulated link knee joint 100 in Fig. 8A (Yb < Ya) .

The sensor 81 may be, for example, the knee angle sensor 58 that detects a position of a magnet embedded in a piston rod or the like with a Hall element or the like and measures an extension/contraction position of the cylinder device 60 and a knee angle substantially on a one-to-one basis with the extension/contraction position, an inertial sensor that detects the posture and motion of the multi-articulated link knee joint 100 by measuring the speed (angular speed) and/or acceleration (angular acceleration) in the translational direction and/or rotational direction of the three axes defining the motion of the multi-articulated link knee joint 100, or a temperature sensor that measures the temperature of the cylinder device 60. The bumper 82 is a shock absorbing component that alleviates an impact when the cylindrical fourth shaft 30 rotates in a cylindrical fifth shaft 31 having a diameter larger than the cylindrical fourth shaft 30.

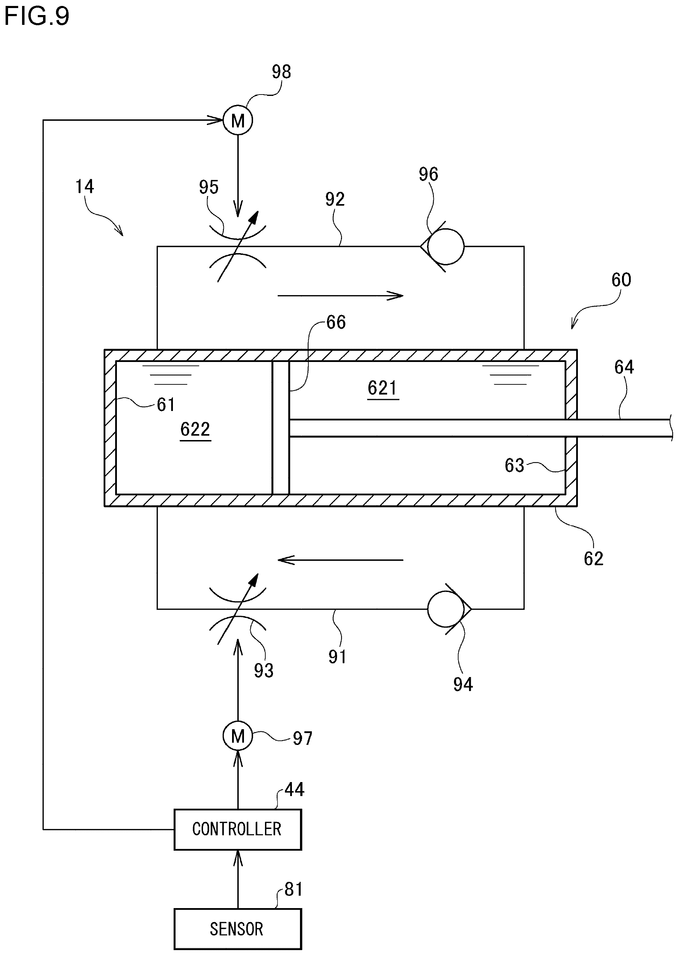

Next, the configuration and operation of the cylinder device 60 will be described. Fig. 9 schematically illustrates the cylinder device 60 of Fig. 7B, etc. The cylinder device 60 includes the cylinder 62, the piston rod 64 that is inserted from one end side (the right end side in Fig. 9 and the upper end side in Fig. 7) of the cylinder 62 and is movable along the longitudinal direction of the cylinder 62 (the left-right direction in Fig. 9 and the vertical direction in Fig. 7), and the piston 66 that is fixed to the piston rod 64 in the cylinder 62 and is slidable in the longitudinal direction along the inner wall of the cylinder 62. The inside of the cylinder 62 is divided by the piston 66 into a first pressure chamber 621 on one end side (the right end side in Fig. 9 and the upper end side in Fig.

7) and a second pressure chamber 622 on the other end side (the left end side in Fig. 9 and the lower end side in Fig. 7). The first pressure chamber 621 is a space defined by the upper surface of the piston 66, the lower surface of the second cylinder wall 63, and the inner wall of the cylinder 62, and the second pressure chamber 622 is a space defined by the lower surface of the piston 66, the upper surface of the first cylinder wall 61, and the inner wall of the cylinder 62. The first pressure chamber 621 and the second pressure chamber 622 are filled with air or oil as a working fluid.

The controller 44 that controls the cylinder device 60 is a pressure drive mechanism that drives the cylinder device 60 to extend and contract by pneumatic pressure or hydraulic pressure. The controller 44 includes a stretching side pressure circuit 91 and a bending side pressure circuit 92 each connected to the cylinder 62. The stretching side pressure circuit 91 and the bending side pressure circuit 92 communicate with the first pressure chamber 621 on one end side and communicate with the second pressure chamber 622 on the other end side, respectively. The stretching side pressure circuit 91 includes a stretching side valve 93 as a valve capable of opening and closing the flow path of the working fluid that generates the rotational resistance of the knee part 10 and a stretching side check valve 94. Opening the stretching side valve 93 allows the working fluid to be distributed in the stretching side pressure circuit 91. The action of the stretching side check valve 94 causes the working fluid to flow only in the direction from the first pressure chamber 621 to the second pressure chamber 622 and not to flow in the opposite direction.

The bending side pressure circuit 92 includes a bending side valve 95 as a valve capable of opening or closing the fluid path of the working fluid for generating the rotational resistance of the knee part 10 and a bending side check valve 96. Opening the bending side valve 95 allows the working fluid to be distributed in the bending side pressure circuit 92. The action of the bending side check valve 96 causes the working fluid to flow only in the direction from the second pressure chamber 622 to the first pressure chamber 621 and not to flow in the opposite direction. The open position of the stretching side valve 93 and the bending side valve 95 can have an arbitrary value between fully opened (the maximum open position) and fully closed (the minimum open position). When each valve is fully closed, the flow of the working fluid is blocked and the rotational resistance of the knee part 10 is maximized, and when each valve is fully opened, the rotational resistance of the knee part 10 is minimized. Specifically, when the stretching side valve 93 is fully closed/fully opened, the rotational resistance to the stretching motion of the knee part 10 is maximized/minimized, and when the bending side valve 95 is fully closed/fully opened, the rotational resistance to the bending motion of the knee part 10 is maximized/minimized.

The stretching side valve 93 and the bending side valve 95 are provided with a stretching side motor 97 and a bending side motor 98 for driving to open and close the stretching side valve and the bending side valve, respectively. The stretching side motor 97 and the bending side motor 98 are controlled by the controller 44, and the open positions of the stretching side valve 93 and the bending side valve 95 are controlled according to the phase of walking of the user of the multi-articulated link knee joint 100. The controller 44 recognizes the phase of walking based on the knee angle measurable by the sensor 81 and the posture and motion of the multi-articulated link knee joint 100. In such walking control or bending and stretching control of the multi-articulated link knee joint 100, it is particularly important to control the bend resistance of the knee part 10 by the bending side valve 95. Specifically, in the Stance phase in which the prosthetic limb is in contact with the ground and is under load, the bend resistance is increased to prevent the knee part 10 from being bent under load (the open position of the bending side valve 95 is minimized). In the Swing phase in which the prosthetic limb leaves the ground and is swung, the bend resistance is decreased (the open position of the bending side valve 95 is maximized) to bend the knee part 10 and prevent the prosthetic limb from touching the ground.

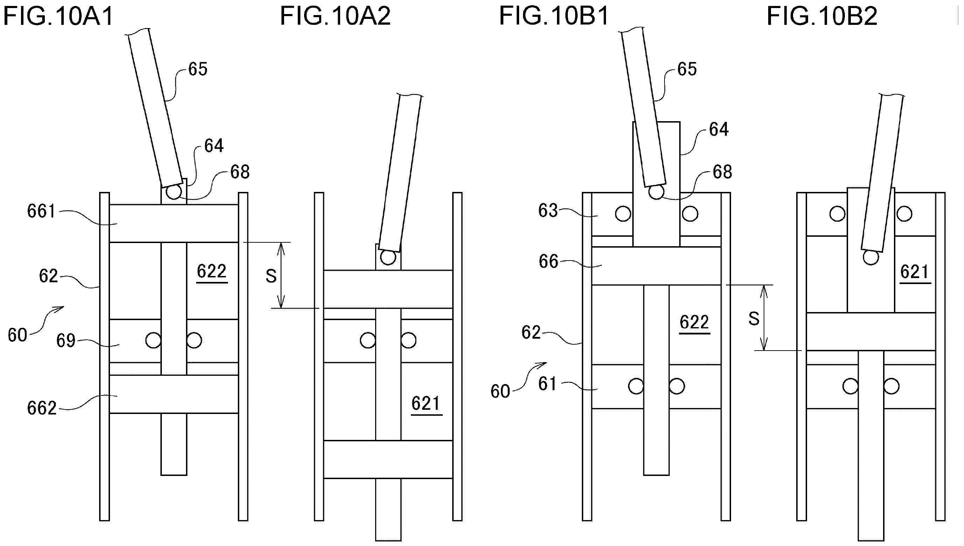

Figs. 10A1, 10A2, 10B1, and 10B2 schematically illustrate a manner in which the cylinder device 60 applies rotational resistance to bending and stretching motions of the multi-articulated link knee joint 100 in Figs. 7A and 7B. Fig. 10B1 illustrates a manner in which the knee part 10 of the multi-articulated link knee joint 100 of Fig. 7B performs the bending motion from the fully stretched state. In this case, the second pressure chamber 622 supporting, from below, the piston 66 that descends with the bending motion applies rotational resistance to the bending motion of the knee part 10. As described above, this rotational resistance can be controlled by the open position of the bending side valve 95. Fig. 10B2 illustrates a manner in which the knee part 10 of the multi-articulated link knee joint 100 of Fig. 7B performs the stretching motion from the fully bent state. In this case, the first pressure chamber 621 supporting, from above, the piston 66 that ascends with the stretching motion applies rotational resistance to the stretching motion of the knee part 10. As described above, this rotational resistance can be controlled by the open position of the stretching side valve 93. A displacement S of the piston 66 between Fig. 10B1 in the fully stretched state and Fig. 10B2 in the fully bent state represents a stroke length, which is a maximum motion amount in the vertical direction.

As shown in Figs. 10B1 and 10B2, when the piston 66 moves from the second cylinder wall 63 side to the first cylinder wall 61 side, that is, when the transition is made from Fig. 10B1 to Fig. 10B2, the connecting part 68 of the connecting rod 65 and the piston rod 64 moves from the thigh joint part 32 side (upper side) toward the lower leg part 12 side (lower side) so as to penetrate the second cylinder wall 63. When the piston 66 is at the limit position on the first cylinder wall 61 side, that is, at the position of Fig. 10B2, the connecting part 68 of the connecting rod 65 and the piston rod 64 is at a corresponding position in the cylinder 62. Alternatively, when the piston 66 is at the limit position of the lower leg part 12 side, the connecting part 68 of the connecting rod 65 and the piston rod 64 is at the position between the second cylinder wall 63 and the piston 66. For convenience of illustration, the connecting rod 65 and the connecting part 68 are shown as being exposed to the outside of the piston rod 64, but in practice, since the connecting rod 65 and the connecting part 68 are provided inside the piston rod 64 as described with reference to Figs. 6A and 6B, the piston rod 64 can slide in the vertical direction while maintaining sealing capability with the second cylinder wall 63.

Fig. 10A1 illustrates a manner in which the knee part 10 of the multi-articulated link knee joint 100 of Fig. 7A performs the bending motion from the fully stretched state. In this case, the second pressure chamber 622 supporting, from below, the upper piston 661 that descends with the bending motion applies rotational resistance to the bending motion of the knee part 10. Fig. 10A2 illustrates a manner in which the knee part 10 of the multi-articulated link knee joint 100 of Fig. 7A performs the stretching motion from the fully bent state. In this case, the first pressure chamber 621 supporting, from above, the lower piston 662 that ascends with the stretching motion applies rotational resistance to the stretching motion of the knee part 10.

As illustrated in Figs. 7B, 10B1, and 10B2, the cylinder 62, the piston 66, the piston rod 64, and the connecting rod 65 connect the thigh joint part 32 and the lower leg part 12 to each other, and constitute a rotational resistance applicator that applies resistance by the working fluid according to the position of the piston 66 in the cylinder 62 to the relative rotation of the thigh joint part 32 and the lower leg part 12. In addition, the functional components such as the controller 44, the sensor 81, the stretching side valve 93, the bending side valve 95, the stretching side motor 97, and the bending side motor 98 illustrated in Fig. 9 control the resistance applied by the rotational resistance applicator according to the phase of walking of the user of the multi-articulated link knee joint 100.

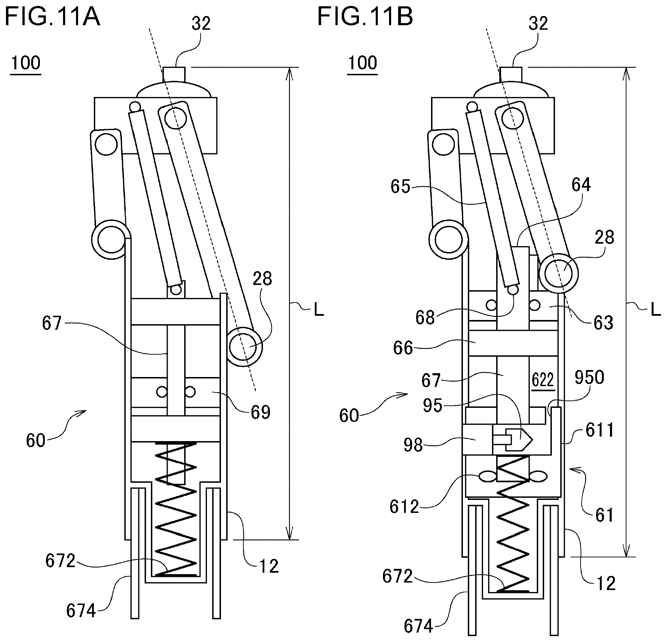

As illustrated in Figs. 7B and 8B, some or all of the functional components including the controller 44 may be disposed in the space above the second cylinder wall 63, or may be stored inside the assembled first cylinder wall 61 as illustrated in Fig. 11B. In this example, the bending side valve 95 and the bending side motor 98 that opens and closes the bending side valve are stored inside the first cylinder wall 61. The flow of the working fluid to and from the second pressure chamber 622 via a flow path 950 is controlled by the control of the open position of the bending side valve 95 by the bending side motor 98, so that it is possible to control the bend resistance applied to the piston 66 and to the knee part 10 by the second pressure chamber 622.

The lower piston rod 67 in Fig. 11A as a comparative example is formed in a columnar shape having a smaller diameter than the extension assist spring 672, whereas the lower piston rod 67 in Fig. 11B is formed in a tubular shape having a larger diameter than the extension assist spring 672. Therefore, in Fig. 11B, the extension assist spring 672 can be inserted into the tubular shaped lower piston rod 67 from below. The upper end of the extension assist spring 672 is fixed inside the lower piston rod 67 to bias the lower piston rod 67 upward. Specifically, the upper end of the extension assist spring 672 is supported from above in contact with a wall (not shown as being hidden behind the storage of the bending side valve 95 and the bending side motor 98 in Fig. 11B) that vertically partitions the inside of the lower piston rod 67 and biases the lower piston rod 67 upward through the wall. As described above, the bending side valve 95 and the bending side motor 98 as functional components are provided inside the first cylinder wall 61 on the lower leg part 12 side and around the extension assist spring 672.

In Fig. 11B, the dimension in the vertical direction of the first cylinder wall 61 in which the functional components are stored become large, but a dimension L in the vertical direction of the entire multi-articulated link knee joint 100 (the distance between the upper end of the thigh joint part 32 and the lower end of the lower leg part 12) can be kept equal to that of Fig. 11A as the comparative example by the configuration in which the connecting part 68 of the connecting rod 65 and the piston rod 64 is provided below the upper end of the piston rod 64 and the configuration in which the fixing point of the extension assist spring 672 and the lower piston rod 67 is provided above the lower end of the lower piston rod 67. On the other hand, as described above, the dimension in the front-rear direction in Fig. 11A of the comparative example becomes larger than that of Fig. 11B due to the third shaft 28 that has to be disposed in front of the cylinder device 60. As described above, according to Fig. 11B, not only the multi-articulated link knee joint 100 can be enhanced in functionality by the functional components stored inside the first cylinder wall 61, but also the multi-articulated link knee joint 100 can be configured compactly in the front-rear direction and the vertical direction.

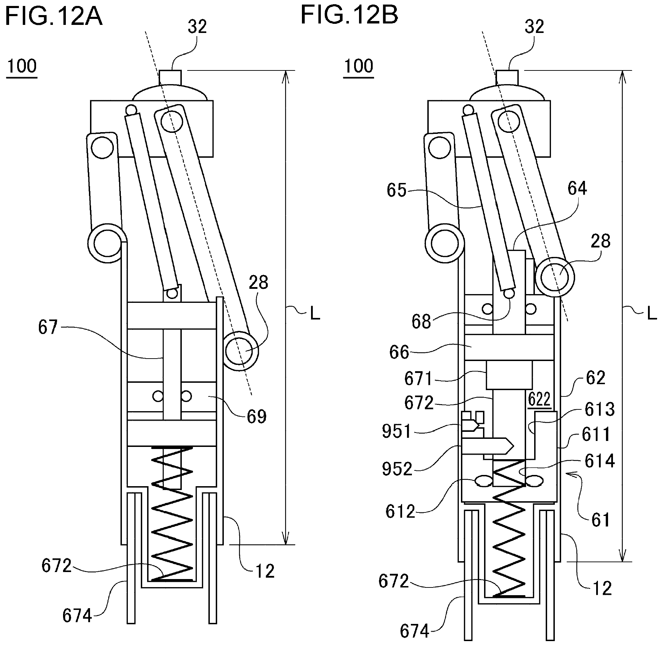

Fig. 12B illustrates an example in which the stepwise control mechanism of the bend resistance by the second pressure chamber 622 is stored inside the assembled first cylinder wall 61 as another example of the functional component. The lower piston rod 67 is provided with a large diameter rod part 671 on the piston 66 side, that is, on the upper side, and a small diameter rod part 672 on the first cylinder wall 61 side, that is, on the lower side. The stepwise control mechanism provided on the first cylinder wall 61 includes a large-diameter part 613 that is continuously provided below the second pressure chamber 622 and into which the large diameter rod part 671 is inserted, a small-diameter part 614 that is continuously provided below the large-diameter part 613 and into which the small diameter rod part 672 is inserted, a first bending side valve 951 that controls the flow of the working fluid in the second pressure chamber 622 and above the large-diameter part 613, and a second bending side valve 952 that controls the flow of the working fluid below the large-diameter part 613. Although not illustrated, a motor for opening and closing the first bending side valve 951 and the second bending side valve 952 may be stored in the assembled first cylinder wall 61.

In the stretched state in which the piston 66 is located upward as illustrated in the drawing, the second pressure chamber 622 and the large-diameter part 613 communicate with each other to form one large pressure chamber. The bend resistance applied to the knee part 10 by this large pressure chamber can be controlled by the open position of the first bending side valve 951 and/or the second bending side valve 952. When the knee part 10 bends from the stretched state of Fig. 12B, the lower piston rod 67 descends, and when the large diameter rod part 671 closes the large-diameter part 613, two pressure chambers are formed vertically. The upper pressure chamber is defined by an outer peripheral surface of the large diameter rod part 671, an inner peripheral surface of the cylinder 62, a lower surface of the piston 66, and an upper surface of the large-diameter part 613 or the first cylinder wall 61, and the lower pressure chamber is defined by an outer peripheral surface of the small diameter rod part 672, an inner peripheral surface of the large-diameter part 613, a lower surface of the large diameter rod part 671, and a bottom surface of the large-diameter part 613. The bend resistance by the upper pressure chamber can be controlled by the open position of the first bending side valve 951, and the bend resistance by the upper pressure chamber can be controlled by the open position of the second bending side valve 952. With such a configuration, since the configuration of the pressure chamber that applies the bend resistance to the knee part 10 can be switched between a case where the bending angle of the knee part 10 is small and a case where the bending angle of the knee part 10 is large, the bend resistance can be finely controlled according to the phase of walking of the user of the multi-articulated link knee joint 100.

As above, the present invention has been described based on the embodiments. The embodiment is intended to be illustrative only and it will be understood by those skilled in the art that various modifications to constituting elements and processes could be developed and that such modifications are also within the scope of the present invention.

In the multi-articulated link knee joint 100 of the embodiments, the cylinder device 60 is fixed to the lower leg part 12, and the connecting rod 65 that connects the piston rod 64 and the thigh joint part 32 is provided. However, the cylinder device 60 may be fixed to the thigh joint part 32, and the connecting rod 65 that connects the piston rod 64 and the lower leg part 12 may be provided. In this case, the top and bottom are reversed from those in Figs. 6A and 6B, and the connecting part 68 of the piston rod 64 and the connecting rod 65 is provided at a position separated by the predetermined distance D from the lower end of the lower leg part 12 side of the piston rod 64 toward the upper end of the piston 66 side. In addition, the top and bottom are reversed from those in Figs. 7A, 7B, 8A, and 8B, and functional components of the multi-articulated link knee joint 100 such as a rotation shaft, a sensor, a bumper, a motor, a control board, and a battery can be disposed in a space below the second cylinder wall 63 on the lower leg part 12 side.

In the multi-articulated link knee joint 100 of Figs. 11B and 12G, the first cylinder wall 61 on the lower leg part 12 side is of an assembled type that can be attached to and detached from the lower side with respect to the cylinder 62, and the functional components of the multi-articulated link knee joint 100 are stored therein. However, the second cylinder wall 63 on the thigh joint part 32 side may be of an assembled type that can be attached to and detached from the upper side with respect to the cylinder 62, and some or all of the functional components of the multi-articulated link knee joint 100 may be stored therein. Since the mass of the distal end side (lower end side) of the lower leg part 12 is reduced by storing the functional components in the upper second cylinder wall 63, the centrifugal force applied to the lower leg part 12 can be reduced to improve the walking stability. The stepwise control mechanism of the bend resistance illustrated in Fig. 12B needs to be provided on the lower first cylinder wall 61, and in a case where a control board or a motor that controls the bend resistance is stored in the upper second cylinder wall 63, wiring or the like that connects both cylinder walls in the vertical direction is provided.

The technical idea of providing the connecting part 68 of the connecting rod 65 at a position separated by the predetermined distance D from the upper end to the lower end of the piston rod 64 illustrated in Figs. 6A, 6B, 7B, and 8B can also be applied to the multi-articulated link knee joint 100 including the two pistons 661 and 662 of Figs. 7A and 8A. Specifically, in Figs. 7A and 8A, the piston rod 64 provided above the upper piston 661 and having a short length in the vertical direction may be formed as a member having a long length in the vertical direction similarly to that of Figs. 7B and 8B, and the connecting rod 65 may be connected to a position separated by the predetermined distance D from the upper end to the lower end thereof.

The technical idea illustrated in Figs. 11B and 12B of storing, inside the cylinder wall, the controller or the functional components to control the resistance applied by the rotational resistance applicator according to the phase of walking of the user can also be applied to the multi-articulated link knee joint 100 including the two pistons 661 and 662 of Figs. 11A and 12A. Specifically, functional components to control the rotational resistance of the multi-articulated link knee joint 100 such as a sensor, a valve, a motor, a control board, a battery, and the like may be stored in the central cylinder wall 69 sandwiched between the upper piston 661 and the lower piston 662 illustrated in Figs. 11A and 12A.

In the embodiments, the multi-articulated link knee joint 100 including the plurality of rotation shafts 24, 26, 28, and 29 and the plurality of front and rear links 20 and 22 has been described, but having at least one rotation shaft may be sufficient, and the front and rear links are not necessarily required. Therefore, the present invention is also applicable to an artificial knee joint having a uniaxial configuration in which the thigh joint part 32 and the lower leg part 12 are simply connected by one rotation shaft.

The functional configuration of each device described in the embodiments can be achieved by hardware resources or software resources or a cooperation of hardware resources and software resources. Processors, ROMs, RAMs, and other LSIs can be used as hardware resources. Programs such as operating systems and applications can be used as software resources.

Among the embodiments disclosed in the present specification, a configuration including a plurality of objects may integrate the plurality of objects, and conversely, a configuration including one object can be divided into a plurality of objects. The present invention may be configured to achieve an object of the invention regardless of whether or not the present invention is integrated.

Among the embodiments disclosed in the present specification in which a plurality of functions are distributed, some or all of the plurality of functions may be aggregated. Conversely, an aggregation of a plurality of functions may be distributed in part of in the entirety. Regardless of whether functions are aggregated or distributed, the functions may be configured to achieve the purpose of the invention.

## Claims

1. An artificial knee joint comprising:
a thigh joint part (32) provided on a thigh part side;
a lower leg part (12) rotatably connected to the thigh joint part (32) via at least one rotation shaft;
a rotational resistance applicator including: a cylinder (62) fixed to one of the thigh joint part (32) and the lower leg part (12) and filled with a working fluid; a piston (66) provided movably between the thigh joint part (32) side and the lower leg part (12) side in the cylinder (62); a cylinder wall (69) provided in the cylinder (62) and defining a motion limit of the piston (66); and a rod part connecting the piston (66) and the other of the thigh joint part (32) and the lower leg part (12), the rotational resistance applicator being structured to apply a resistance by the working fluid according to a position of the piston (66) in the cylinder (62) to relative rotation of the thigh joint part (32) and the lower leg part (12); and
a functional component that is stored inside the cylinder wall (69) and controls the resistance applied by the rotational resistance applicator according to a phase of walking of a user.

2. The artificial knee joint according to claim 1,
wherein the functional component includes a motor that drives to open and close a valve capable of opening and closing a flow path of the working fluid connected to at least one of a first pressure chamber (621) of one side of the thigh joint part (32) and lower leg part (12) and a second pressure chamber (622) of the other side of the thigh joint part (32) and the lower leg part (12), the first pressure chamber (621) and the second pressure chamber (622) being formed by partitioning inside of the cylinder (62) by the piston (66) or the cylinder wall (69).

3. The artificial knee joint according to claim 1 or 2, wherein
the cylinder wall (69) comprises a first cylinder wall (61) on one side of the thigh joint part (32) and the lower leg part (12), and a second cylinder wall (63) on the other side of the thigh joint part (32) and lower leg part (12),
the piston (66) is provided movably between the first cylinder wall (61) and the second cylinder wall (63), and
the functional component is stored inside of at least one of the first cylinder wall (61) and the second cylinder wall (63).

4. The artificial knee joint according to claim 3, wherein
the rod parts comprises a large diameter rod part (671) on the piston (66) side and a small diameter rod part (672) on a cylinder wall (69) side in which the functional component is stored, and
the functional component includes a large-diameter part (613) into which the large diameter rod part (671) is inserted and a small-diameter part (614) into which the small diameter rod part (672) is inserted.

5. The artificial knee joint according to claim 3 or 4, wherein
the rod part comprises a tubular shaped piston rod (64) penetrating a cylinder wall (69) on the lower leg part (12) side,
the artificial knee joint further comprising an extension assist spring whose upper end is fixed inside the piston rod (64) and that biases the piston rod (64) upward.

6. The artificial knee joint according to claim 5, wherein the functional component is provided inside a cylinder wall (69) on the lower leg part (12) side and around the extension assist spring.

7. The artificial knee joint according to any of claims 3 to 6, wherein at least one of the first cylinder wall (61) and the second cylinder wall (63) is attachable to and detachable from the cylinder (62).

8. The artificial knee joint according to claim 7, wherein a cylinder wall (69) on the lower leg part (12) side is attachable to and detachable from the cylinder (62).

9. The artificial knee joint according to claim 8, wherein the rotation shaft is provided above a cylinder wall (69) on the thigh joint part (32) side.

10. The artificial knee joint according to claim 8 or 9, wherein the functional component is provided above a cylinder wall (69) on the thigh joint part (32) side.

11. The artificial knee joint according to claim 1 or 2, wherein
the cylinder wall (69) vertically partitions inside of the cylinder (62),
the piston (66) comprises two pistons provided movably and connected to each other with the cylinder wall (69) interposed therebetween from above and below, and
the rod part couples the piston (66) on the other side of the thigh joint part (32) and the lower leg part (12) and the other.

12. The artificial knee joint according to claim 1, wherein the cylinder wall (69) defines a motion limit of the other side of the thigh joint part (32) and the lower leg part (12) of the piston (66), further comprising:
a piston rod (64) extending from the piston (66) toward the other of the thigh joint part (32) and the lower leg part (12) and penetrating the cylinder wall (69); and
a connecting rod (65) that connects the piston rod (64) to the other of the thigh joint part (32) and the lower leg part (12) and is connected to the piston rod (64) at a connecting part (68) that is separated by a predetermined distance or more from the other end of the other side of the thigh joint part (32) and the lower leg part (12) on the piston rod (64) toward one end on the piston (66) side,
wherein
when the piston (66) is at a limit position of one side of the thigh joint part (32) and the lower leg part (12), the connecting part (68) of the connecting rod (65) and the piston rod (64) is positioned between the cylinder wall (69) and the piston (66).

13. The artificial knee joint according to claim 12, wherein
the piston rod (64) includes a tubular part, and
the connecting part (68) of the connecting rod (65) and the piston rod (64) is provided inside the tubular part of the piston rod (64).
